# EUROPEAN PATENT SPECIFICATION

(11) **EP 4 052 032 B1**
(45) Date of publication and mention of the grant of the patent: **19.03.2025**
(21) Application number: 20803234.2
(22) Date of filing: 26.10.2020
(51) Int. Cl.: G01N 33/24, G01N 27/12, G01N 1/22

(54) **SYSTEM FOR ANALYSING VOLATILE ORGANIC COMPOUNDS IN SOIL**
SYSTEM ZUR ANALYSE VON FLÜCHTIGEN ORGANISCHEN VERBINDUNGEN IM BODEN
SYSTÈME D'ANALYSE DE COMPOSÉS ORGANIQUES VOLATILS DANS LE SOL

(30) Priority: 29.10.2019 GB 201915639
(43) Date of publication of application: 07.09.2022
(60) Divisional application: 25157178.2
(73) Proprietor: P.E.S. Technologies Limited, Diss Norfolk IP22 4QA (GB)
(72) Inventor: BAILEY, Jim, Diss Norfolk IP22 4QA (GB); BAILEY, Graham, Diss Norfolk IP22 4PL (GB)
(74) Representative: Windsor, Louise
(86) International application number: PCT/GB2020/052707
(87) International publication number: WO 2021/084235

(56) References cited:
- WO-A1-2018/173060
- CN-A- 107 991 352
- US-A1- 2005 150 778
- US-A1- 2012 156 099
- GUTIERREZ F J ET AL: "Integrated sensors for monitoring contaminant gases in atmospheres and soils", INDUSTRIAL ELECTRONICS, 1997. ISIE '97., PROCEEDINGS OF THE IEEE INTER NATIONAL SYMPOSIUM ON GUIMARAES, PORTUGAL 7-11 JULY 1997, NEW YORK, NY, USA,IEEE, US, vol. 1, 7 July 1997 (1997-07-07), pages SS113 - SS115, XP010265149, ISBN: 978-0-7803-3936-1, DOI: 10.1109/ISIE.1997.651745

## Description

The present invention relates to a system for analysing volatile organic compounds (VOCs) in soil and/or gases emitted from soil and a method of analysing soil.

### BACKGROUND TO THE INVENTION

It is known that an important part of optimising growing crops in soil is ensuring the health and fertility of the soil. Current soil analysis tools require samples to be sent to a testing centre and return results in a number of days. This delay means the user, such as a farmer, is not able to quickly analyse the health of their soil.

Crop rotation is known to improve yields of crops. Further, it is known to treat land to improve the fertility of the soil, such as by providing fertilisers, biomass, fungicides, fungi, bactericides and bacteria.

It is known to analyse carbon dioxide emissions from soil, however, this does not indicate the type of microorganisms that are present in the soil and only indicates the degree of anaerobic respiration.

Soil microorganisms produce a range of volatile organic compounds (VOCs). The composition of VOC emissions may provide general information about the structure of soil microbial communities.

It is known to use electronic noses to assess the VOCs from various compositions. Current "electronic noses" that are reliable typically use impedance spectroscopy to analyse the VOCs. This typically requires a three-terminal structure with a source electrode, a gate electrode and a drain electrode. The impedance is measured across a band of frequencies. However, impedance spectroscopy requires mathematical modelling to analyse the curve.

WO95/32422 discloses a sensor comprising a plurality of electric circuit elements sensitive to different substances, an electric circuit including said elements and a circuity in said circuit responsive to the condition of said circuit elements and connected to an output device, said circuitry being adapted to actuate the output device in response to one or more combination of conditions of the individual circuit elements. The equipment is used in the detection of gases arising from microbiological activity to detect certain pathological conditions such as necrosis or infection in wounds, or in fermentation monitoring in the brewing industry. An output device may comprise a two-state indicator (on or off) to show that one or more of several gases has been detected, but not precisely what has been detected.

WO 95/25268 discloses a testing vessel for analysing a sample, which is placed in a first compartment that can be purged with a gas prior to taking a measurement in order to ensure that the atmosphere within the chamber is of a known composition. The sample can be a liquid such as perfume or drinks or a solid such as foodstuffs. Sensors capable of detecting gas, vapours or other volatile materials, e.g. using a semi-conductive polymer and a pair of electrical contacts, are placed in a second compartment that can be independently purged and means is provided for establishing communication between the compartments.

A system for analysing VOCs in soil, comprising an array of semiconducting metal oxide sensors, is described by Gutierrez et al. in Proceedings of the IEEE International Symposium on Industrial Electronics, 7-11 JULY 1997, pages SS113-SS115, DOI: 10.1109/ ISIE.1997.651745, ISBN: 978-0-7803-3936-1.

There is a need for an efficient way for analysing the VOCs in soil. There is a need for a handheld device which allows a user to quickly obtain an analysis of soil health. There is a need for cost effective sensor strips. There is a need for an easy method of analysing soil health. There is a need for a battery powered analysis apparatus that allows soil to be analysed in the field, rather than requiring samples to be taken to a separate analysis area. There is a need for a method of increasing the yield of crops. There is a need for real time advice on how to improve soil health. There is a need for a low powered device for analysing soil. There is a need for a simple way to analyse the data from a sensor. There is a need for an apparatus with minimal components.

It is, therefore, an object of the present invention to seek to alleviate the above identified problems.

### SUMMARY OF THE INVENTION

According to a first aspect of the present invention, there is provided a system for analysing volatile organic compounds (VOCs) in soil comprising an apparatus and a soil VOC sensor strip,
wherein the apparatus comprises a sampling chamber for receiving soil, a sensor strip aperture in the sampling chamber for positioning the sensor strip in fluid communication with the sampling chamber, a power source and an electrical resistance detector,
wherein the sensor strip is electrically connectable to the power source and the electrical resistance detector; and
wherein the system further comprises a display and/or data storage, wherein the electrical resistance detector is configured to transmit output to the display and/or the data storage and wherein the data storage is configured to send the output for comparison with a known dataset and to store a comparison output, and the display is configured to display the comparison output,
characterised in that the sensor strip comprises a flexible substrate with a first surface and an array of semiconductor polymer sensors arranged on the first surface, wherein each of the semiconductor polymer sensors comprises a pair of electrodes, wherein the pair of electrodes comprises a first electrode and a second electrode, wherein a semiconductor polymer is disposed between the first electrode and the second electrode.

In another aspect of the present invention, there is provided a method of analysing soil comprising:
a) providing an apparatus, wherein the apparatus comprises a sampling chamber, a sensor strip aperture in the sampling chamber, a power source and an electrical resistance detector; a display and/or data storage;
b) providing a soil volatile organic compound (VOC) sensor strip,
   wherein the sensor strip comprises a flexible substrate with a first surface and an array of semiconductor polymer sensors arranged on the first surface,
   wherein each of the semiconductor polymer sensors comprises a pair of electrodes, wherein the pair of electrodes comprises a first electrode and a second electrode, wherein a semiconductor polymer is disposed between the first electrode and the second electrode;
c) providing a soil sample,
d) positioning the sensor strip over the sensor strip aperture, wherein one or each of the semiconductor polymer sensors are in fluid communication with the sampling chamber, wherein each pair of electrodes is electrically connected to the power source and the electrical resistance detector,
e) positioning the soil sample in the sampling chamber,
f) actuating the power source to supply electricity to the sensor strip, and
g) detecting the electrical resistance of one or each of the semiconductor polymer sensor using the electrical resistance detector to give an output.
h) transmitting output from the electrical resistance detector to the display and/or the data storage; sending the output for comparison with a known dataset and displaying a comparison output on the display and/or storing the comparison output on the data storage.

### BRIEF DESCRIPTION OF THE DRAWINGS

Figure 1 shows the apparatus of the invention.
Figure 2 shows the system of the invention
Figure 3 shows the sensor strip of the invention
Figure 4 shows a magnified semiconductor polymer sensor of the invention.
Figure 5 shows a cross section of a semiconductor polymer sensor of the invention.
Figure 6 shows a cross section of a soil volatile organic compound (VOC) impedance spectroscopy sensor strip.
Figure 7 shows an embodiment of the apparatus of the invention having a cassette chamber and Figure 8 shows a cassette housing to be installed in the apparatus of Figure 7.
Figure 9 shows a first sample sensor response.
Figure 10 shows a second sample sensor response.

### DETAILED DESCRIPTION

The present invention relates to a system for analysing volatile organic compounds (VOCs) in soil comprising an apparatus and a soil VOC sensor strip,
wherein the apparatus comprises a sampling chamber for receiving soil, a sensor strip aperture in the sampling chamber for positioning the sensor strip in fluid communication with the sampling chamber, a power source and an electrical resistance detector,
wherein the sensor strip comprises a flexible substrate with a first surface and an array of semiconductor polymer sensors arranged on the first surface, wherein each of the semiconductor polymer sensors comprises a pair of electrodes, wherein the pair of electrodes comprises a first electrode and a second electrode, wherein a semiconductor polymer is disposed between the first electrode and the second electrode, and
wherein the sensor strip is electrically connectable to the power source and the electrical resistance detector,
wherein the system further comprises a display and/or data storage, wherein the electrical resistance detector is configured to transmit output to the display and/or the data storage, and wherein the data storage is configured to send the output for comparison with a known dataset and to store a comparison output, and the display is configured to display the comparison output.

The system of the invention provides an efficient way for analysing the VOCs in soil and/or gases emitted from soil. The sensor strips are efficient to manufacture and are low cost as the substrate is a flexible substrate. These are much lower cost than manufacturing silicon substrates such as those used in the art. A further advantage is that the system has a two-terminal electrode set up and measures resistance. There is no need for a mathematical model to analyse the resistance, unlike the more complicated analysis process used in impedance spectroscopy, this means that the data of the invention is less expensive computationally to analyse, as it does not require a mathematical model. In particular, resistance data may be directly fed into a machine learning algorithm, whereas impedance data needs to be first fit into a mathematical model, and then fed into a machine learning algorithm. This additional step of fitting impedance data to a mathematical model requires more complicated electronics and more computing power, than is required for using resistance data. Further, as the resistance is measured, there is not a risk that the mathematical model is incorrect. Further, the cost is reduced by only requiring two electrodes as this is more efficient to manufacture than a three-terminal electrode set up. In particular, it is possible to print two electrodes at the same stage of the printing process. A three terminal electrode structure requires much greater precision in printing as it is necessary for several stages of printing to be used to print the whole structure and it cannot be printed at one position. This increases the length of the production line as the flexible substrate is passed through different print stages. Further, it is important in a three-terminal structure that there is no overlap of the gate electrodes with the other electrodes, which makes registration much harder. Further, only requiring two-terminal electrodes reduces the thickness of the sensor, and the amount of conductive material, such as metals used to make the electrodes and reduces the number of layers in the sensor. A further advantage of a two-terminal structure is that less terminals are used; therefore, the reliability is higher, the production cost is lower, and the complexity is lower than a three-terminal structure. The apparatus in the system has minimal components. Further the use of an electrical resistance detector reduces the level of voltage required, particularly compared to impedance spectroscopy which requires a high voltage to carry out the analysis. The lower voltage makes it easier to provide a battery powered device, in particular it simplifies the electronics. Further, by comparing the output with a known data set gives the user information about the current condition of the soil. The comparison output is preferably advice on how to improve the soil.

Preferably the system of the present invention does not use impedance spectroscopy.

Preferably fluid communication means that fluid can move from the sampling chamber to the sensor strip, preferably gases, such as VOCs, can move from the sampling chamber to the sensor strip.

It is understood that the system for analysing volatile organic compounds (VOCs) in soil of the present invention, which comprises a soil VOC sensor strip, is also or alternatively suitable for analysing gases emitted from soil. The soil VOC sensor strip of the present invention is also suitable for sensing gases emitted from soil.

Preferably, the power source is a battery. This allows the system to be used at any location, and in particular, it allows the system to be used in a field, such that the analysis can be carried out where the soil sample is collected. This allows the user to get the results at a predetermined location and they do not need to collect and label soil samples for later analysis.

Preferably, the battery is rechargeable. This is more environmentally friendly than disposable batteries.

Preferably the battery is solar-powered. This gives the user more flexibility to carry out experiments without needing to connect the battery to another power source to recharge it, or to replace the battery.

Preferably, the apparatus comprises a GPS locator. This allows the location that the sample is collected from to be easily recorded.

Preferably, the apparatus further comprises a housing. Preferably, the housing comprises the sampling chamber. Preferably, the housing further comprises the electrical resistance detector and/or the power source. Preferably, the housing further comprises the electrical resistance detector and the power source. Preferably the housing is waterproof. The housing is a suitable way to hold the apparatus together so that it can be transported. Further, the housing protects parts of the apparatus from the elements, such as rain. In particular, it is advantageous for the electronic parts, such as the power source and the electrical resistance detector to be protected from water.

Preferably, the display and/or the data storage is connectable to the electrical resistance detector via Bluetooth, Wi-Fi, a mobile wireless communication system a cable or direct electrical connection, preferably via Bluetooth, Wi-Fi or a mobile wireless communication system, preferably via Bluetooth. This allows the output to be displayed or stored.

Preferably, the display and/or data storage is a personal electronic device (PED), preferably the PED is a smart phone, a tablet, a mobile phone, a laptop or a notebook, preferably a smart phone. It is particularly useful if the PED is handheld.

Preferably, the electrical resistance detector is any detector measuring an output such that the resistance of the semiconductor polymer can be measured or calculated. The electrical resistance detector may measure current and voltage which allows the resistance to be calculated. The electrical resistance detector is preferably an ohmmeter. Preferably, impedance is not measured and/or analysed.

Preferably, the sampling chamber has an open position and a closed position. This allows a soil sample to be added into the sampling chamber and then allows the soil sample to be held in a closed position for analysis. Preferably the closed position means that the only substantial opening in the sampling chamber is the sensor strip aperture. This allows the VOCs to be contained in the sampling chamber and analysed via the sensor strip aperture.

Preferably, the sampling chamber is a drawer, and the drawer is movable from the open position to the closed position. This is a convenient way to introduce the soil sample for analysis.

Preferably, the apparatus further comprises a switch, wherein actuating the switch turns on the power source or wakes the power source from sleep mode. This allows the user to control when the analysis of the soil starts. The switch may be actuated by the user, such as by physically pressing a switch, or activating it electronically, such as via a display connectable to the apparatus.

Preferably the switch is a microswitch. This is a reliable way of actuating the power source.

Preferably, the switch is actuated when the sampling chamber is in the closed position. This allows the detection to start as soon as the soil sample is in position.

Preferably the switch is actuated when the drawer is moved to the closed position. Preferably the switch comprises a magnet and a magnetic sensor and the magnetic sensor turns on the power source or wakes up the power source from sleep mode. This provides a convenient way of starting the analysis.

Preferably, the power source is turned off after a predetermined period of time. This allows the analysis to be carried out for the desired period of time. This reduces power usage so that voltage is only applied to the sensor strip when it is needed.

Preferably, the power source is automatically turned off or is put into sleep mode after a predetermined period of time, preferably when the measurement is finished. This reduces the input required from the user and helps produce consistent output for later analysis.

Preferably, the predetermined period of time is at least about 30 seconds, preferably about 30 seconds to about 60 minutes, preferably about 30 seconds to about 30 minutes, preferably about 1 minute to about 15 minutes, preferably about 1 minute to about 10 minutes, preferably about 2 minutes to about 8 minutes, preferably about 3 minutes to about 6 minutes. Whilst the analysis can be carried out for long periods of time, it is preferable that the user gets quick feedback, such as within a few minutes, such as less than 10 minutes. This is particularly advantageous because the user is given advice about the soil in a timely manner.

Preferably, the apparatus further comprises a sensor strip securing device. Preferably, the sensor strip securing device is attached to the housing. Preferably, the sensor strip securing device is pivotably attached to the housing. Preferably wherein the sensor strip securing device is a lever movable from an open position to a closed position. It is advantageous to be able to hold the sensor strip in position. Typically, the system will be used outside, such as in a field where it will be exposed to the elements, such as to the wind. It is advantageous for the sensor strip to be held in position such that it does not substantially move or blow away. The aim of this is to ensure that each of the first electrode and the second electrode are connected to the appropriate electrical connections within the apparatus.

Preferably, the apparatus further comprises a sensor strip positioning device. Preferably the positioning device is a recess in the apparatus that is sized and shaped to receive the sensor strip. An advantage of this is to ensure that each of the first electrode and the second electrode are connected to the appropriate electrical connections within the apparatus.

Preferably, the apparatus further comprises a sensor strip location detector. This allows the apparatus to check whether the sensor strip has been correctly position, and that the or each of the first electrode and the second electrode have been electrically connected to the electrical resistance detector. Preferably this is achieved by a microswitch, a magnetic switch, an optical sensor, a circuit formed when the sensor strip is placed in position or a short circuit formed when the sensor strip is placed in position, or any combination of two or more thereof, preferably a circuit or short circuit formed when the sensor strip is placed in position.

Preferably, the apparatus comprises a heater for heating the sampling chamber.

Preferably, the apparatus further comprises a sensor strip storage device; preferably wherein the sensor strip storage device is substantially sealed; and/or wherein the sensor strip storage device is a cassette.

Preferably, the apparatus further comprises at least one movement mechanism for automatic replacement and/or positioning of the sensor strip in fluid communication with the sampling chamber; preferably, wherein the movement mechanism comprises at least two spools for moving the sensor strip therebetween and across the sampling chamber; optionally, wherein the movement mechanism comprises a stepper motor and/or a sensor strip positioning device.

The automatic replacement of the sensors protects the sensor from contamination prior to use or during replacement and so reduces the need for single-use plastic packaging for each sensor. Further the automatic replacement and secure storage of the sensors reduces the complexity of the apparatus for users to reduce any possible errors introduced by incorrect insertion of the sensors into the apparatus.

Preferably, the sensor strip storage device is sealed containing at least one gas to prevent ingress of air, moisture or contaminants.

Preferably, the apparatus is a handheld apparatus. This means it is easy for the user to transport and carry the apparatus to a desired place of use. It is particularly advantageous that the apparatus can be used at any location, and in particular outside, such as in a field.

Preferably, the sampling chamber has a maximum capacity in a range of about 10 cm³ to about 300 cm³, preferably in a range of about 50 cm³ to about 200 cm³, preferably in a range of about 70 cm³ to about 150 cm³. The maximum capacity is preferably the volume of soil that can be put into the sampling chamber in the open position, which allows the sampling chamber to be moved to the closed position. In practice, the user does not need to completely fill the sampling chamber, however it is desirable that as much soil as fits the sampling chamber is included to maximise the VOC levels.

Preferably, the distance between the level of the soil when the sampling chamber is filled to maximum capacity, and the sensor strip, is in the range of about 0.5 cm to 5 cm, preferably about 1 cm to about 3 cm. It is preferable that there is some space between the level of the soil and the sensor strip to allow the VOC to reach each of the semiconductor polymers. Preferably there is not excessive space as this will dilute the concentration of the VOCs and increase the measurement time.

Preferably the soil does not come into contact with the sensor strip. This reduces contamination of the semiconductor polymer sensors and ensures the greatest surface area of the sensor is available to the VOCs.

Preferably the semiconductor polymer sensors are positioned in relation to the soil sample such that the VOC can reach the sensor in sufficient quantity in a reasonable amount of time. Preferably, the headroom when the sampling chamber is filled to maximum capacity and when the sampling chamber is in the closed position has a volume in the range of about 3 cm³ to about 50 cm³, preferably about 10 cm³ to about 30 cm³. The headroom is the volume of space in the sampling chamber which is above the soil level. Such volumes are appropriate for allowing the VOCs to be emitted by the soil, whilst keeping the concentrations high enough to be read.

Preferably the sensor strip aperture is positioned substantially above the soil sample. Preferably the sensor strip is positioned with its first surface facing substantially downwards towards the soil sample. This allows the VOCs to rise and come into contact with the semiconductor polymer sensors.

Preferably one or each of the pair of electrodes are in the form of interdigitated fingers or concentric spirals, preferably in the form of interdigitated fingers. Such arrangements reduce the area covered by the electrodes and allows efficient use of space. The form of interdigitated fingers is particularly preferred as these can be easily printed. In particular, the interdigitated fingers preferably have substantially perpendicular turns between subsequent fingers and the fingers are substantially straight. This makes them easier to print.

Preferably, one or each of the first electrode and/or one or each of the second electrode have a sheet resistivity of less than about 30 Ohm/Square, preferably less than about 20 Ohm/square.

Preferably, one or each of the first electrode and/or one or each of the second electrode comprises a metal, a metal oxide, an electrically conductive polymer, graphene or carbon, preferably, silver, gold, copper, zinc, carbon, graphene nanoplatelets, indium tin oxide, poly(3,4-ethylenedioxythiophene) polystyrene sulfonate (PEDOT:PSS), 3,4-ethylenedioxythiophene or a derivative thereof or poly(3,4-ethylenedioxythiophene or a derivative thereof or any combination of two or more thereof, preferably silver, gold, copper, zinc or carbon, preferably silver, gold copper or zinc or any combination of two or more thereof, preferably silver, gold or copper or any combination of two or more thereof, preferably silver and/or gold, preferably silver. Silver, gold and copper are particularly suited to printing. Silver is preferred for ease of use and cost.

Preferably, the distance between the first electrode and the second electrode of one or each of the pair of electrodes is in the range of about 0.1 µm to about 40 µm, preferably in the range of about 1 µm to about 30 µm, preferably in the range of about 4 µm to about 20 µm, preferably in the range of about 5 µm to about 15 µm. These distances are suitable for detecting the electrical resistance in the present invention. The small distances are particularly preferred as they increase the sensitivity of the system.

Preferably, one or each of the first electrode and/or one or each of the second electrode are printed, preferably flexography printed, gravure printed, offset printed, screen printed or digital printed, preferably flexography printed, or gravure printed, most preferably flexography printed. This allows the sensors to be consistently reproduced in a high throughput manner. It is possible to use a different printing mechanism for each first and each second electrode, however for an efficient manufacturing process, preferably each electrode is printed using the same printing process.

Preferably, the length of one or each of the first electrode and/or one or each of the second electrode is in the range of about 1 cm to about 50 cm, preferably in the range of about 2 cm to about 30 cm, preferably in the range of about 3 cm to about 20 cm, preferably in the range of about 5 cm, to about 15 cm. Such lengths are suitable for use in the present invention. This balances the desire for long electrodes with the desire for these to take up the minimum space. It will be appreciated that the length of the electrode is the resistive length, that is the length of the electrode if it were arranged in a single substantially straight line.

Preferably, the width and thickness of one or each of the first electrode and/or one or each of the second electrode is each independently in the range of about 3 nm to about 1.5 µm, preferably in the range of about 5nm to about 1 µm, preferably in the range of about 10 nm to about 0.5 µm, preferably in the range of about 100 nm to about 300 nm. Such widths and thicknesses are suitable for allowing the electrical resistance to be measured.

Preferably each semiconductor polymer sensor does not comprise a third electrode, in particular, each semiconductor polymer sensor does not comprise a gate electrode. Preferably each semiconductor polymer sensor does not have a field effect transistor.

In an alternative embodiment, each semiconductor polymer sensor comprises three electrodes. This may increase the sensitivity of the sensor as the presence of a third electrode, and in particular a gate electrode reduces the resistance of the sensor. In this embodiment, the resistance is measured, and impedance is not measured.

Preferably, one or each of the semiconductor polymers is an organic semiconductor polymer, preferably a conjugated polymer, preferably a doped conjugated polymer. Preferably, one or each of the semiconductor polymers comprises an organic conjugated polymer with side chains, wherein the side chains may be conjugated or unconjugated. Preferably, the side chains comprise carbon atoms and optionally heteroatoms, such as oxygen, nitrogen or sulphur, or a combination of two or more thereof. Such semiconductor polymers are particularly suitable for use in the invention.

Preferably, one or each of the semiconductor polymers has a molecular weight of about 10,000 to about 500, 000. Such molecular weights are easily printed.

Preferably, at least two of the semiconductor polymers comprises a different semiconductor polymer, preferably wherein each of the semiconductor polymers comprises a different semiconductor polymer. It is an advantage that the semiconductor polymers are different so that they have a different reaction to the VOCs. This allows multiple fingerprints to be produced for analysis.

Preferably, one or each of the semiconductor polymers has a thickness of about 1 nm to about 100 µm, preferably about 1 nm to about 1 µm, preferably about 2 nm to about 100 nm, preferably about 3 nm to about 50 nm, preferably about 10 nm to about 30 nm. Such thicknesses are sufficient for the present invention.

Preferably, one or each of the semiconductor polymers are printed, preferably digital printed flexography printed, gravure printed, screen printed or offset printed, preferably digital printed, preferably inkjet printed. This allows the sensors to be consistently reproduced in a high throughput manner. Preferably each semiconductor polymer is printed in the same way for process efficiency. However, it is possible to use different printing techniques for different semiconductor polymers

Suitable semiconductor polymer inks that can be used in the present invention may be purchased such as from Sigma Aldridge or Ossila. Preferably the semiconductor polymer inks comprise, F8BT - poly(9,9-dioctylfluorene-alt-benzothiadiazole), F8T2 - poly(9,9-dioctylfluorene-alt-bithiophene), PBDD4T-2F - poly[[5,7-bis(2-ethylhexyl)-4,8-dioxo-4H,8H-benzo[1,2-c:4,5-c']dithiophene-1,3-diyl][3,3‴-bis(2-ethylhexyl)-3",4'-difluoro[2,2':5',2":5",2‴-quaterthiophene]-5,5‴-diyl]], PDBPyBT - poly(2,5-bis(2-octyldodecyl)-3,6-di(pyridin-2-yl)-pyrrolo[3,4-c]pyrrole-1,4(2H,5H)-dione-alt-2,2'-bithiophene), PNDI(2HD)2T - poly{[N,N'-bis(2-hexyldecyl)naphthalene-1,4,5,8-bis(dicarboximide)-2,6-diyl]-alt-5,5'-(2,2'-bithiophene)}, PTB7 - poly[[4,8-bis[(2-ethylhexyl)oxy]benzo[1,2-b:4,5-b']dithiophene-2,6-diyl][3-fluoro-2-[(2-ethylhexyl)carbonyl]thieno[3,4-b]thiophenediyl]], or PolyTPD - poly[N,N'-bis(4-butylphenyl)-N,N'-bisphenylbenzidine], or any combination of two or more thereof.

Preferably, at least one of the semiconductors polymers is doped. This provides an efficient way to adjust the properties of the semiconductor polymer. This has the advantage that one ink may be purchased and then the properties changed by adding one or more dopants to the ink, preferably in amount of about 0.01 wt% to about 25 wt%, preferably 0.1 wt% to 5 wt%. A doped polymer semiconductor ink is considered different to the original polymer semiconductor ink. If a different dopant is used, again this will be a different polymer semiconductor ink.

Dopants that can be used to change the properties of the semiconductor polymers that can be used in the present invention may be purchased such as from Sigma Aldridge or Ossila. Preferably the dopant comprises tris(2-methoxyethoxy)(vinyl)silane, 2,3,5,6-tetrafluoro-7,7,8,8-tetracyanoquinodimethane, 9,10-bis[N,N-di-(*p*-tolyl)-amino]anthracene, 4,4'-bis(2,2-diphenylvinyl)biphenyl, 7,7,8,8-tetracyanoquinodimethane, N,N'-dimethylquinacridone, 4,4'-bis(2,2-diphenylvinyl)biphenyl, lithium-8-hydroxyquinolinolate or tris(2-(1H-pyrazol-1-yl)pyridine)cobalt(III) tri[hexafluorophosphate] or any combination of two or more thereof.

Preferably, at least one semiconductor polymer sensor can show the presence of alcohols, aldehydes, arsenics, ketones, benzenoids, carboxylic acids, ethers, esters, hydrocarbons, terpenes, alkenes, thiofurans, alkynes, bromides, nitriles, chlorides, pyrazines and sulphides, or any combination of two or more thereof, preferably esters, aldehydes, aldehyde derivatives, tertiary alcohols or terpenes, or any combination of two or more thereof, preferably propanoic acid and butyric acid, furfural, 5-(hydroxymethyl)furfural, (+/)-Geosmin and 2-methylisoborneol, the terpene, β-caryophyllene, isopropanol, acetone, toluene or any combination of two or more thereof. Preferably the semiconductor polymers are selected to show a reaction to different VOCs such that a set of outputs can provide advice based on a number of VOCs.

Preferably at least one of the semiconductor polymer sensors can show the presence of (+/-)-Geosmin and/or 2-methylisoborneol. Preferably, at least one of the semiconductor polymer sensors can show the presence of actinobacteria, preferably by detecting the presence of (+/-)-Geosmin and/or 2-methylisoborneol.

Preferably at least one of the semiconductor polymer sensors can show the presence of alkenes, ketones, pyrazines or terpenes or any combination of two or more thereof. Preferably, at least one of the semiconductor polymer sensors can show the presence of bacteria, preferably by detecting the presence of alkenes, ketones, pyrazines or terpenes or any combination of two or more thereof.

Preferably at least one of the semiconductor polymer sensors can show the presence of benzenoids, aldehydes, arsenics, chlorides, nitriles, thiofurans, alkines or bromides or any combination of two or more thereof. Preferably, at least one of the semiconductor polymer sensors can show the presence of fungi, preferably by detecting the presence of benzenoids, aldehydes, arsenics, chlorides, nitriles, thiofurans, or bromides or any combination of two or more thereof.

Preferably the substrate is planar.

Preferably flexible means that the substrate can be provided on a roll, preferably wherein the substrate is printable using web-fed presses. Preferably flexible means that the substrate has a thickness of less than 250 µm, preferably less than 150 µm. An advantage of the substrate being flexible is that during production of the sensor strip, a roll of flexible substrate can be used, and the printed flexible substrate can be rolled and stored until it is needed to be divided into individual sensors. Further the weight of a flexible substrate is reduced which is better for the environment as less polymeric material is used per sensor strip.

Preferably, the sensor strip comprises a backing layer on a second surface of the flexible substrate, wherein the second surface opposes the first surface, preferably wherein the backing layer comprises paper, card, or a polymeric film, preferably paper. The advantage of a backing layer is that it gives structural rigidity to the sensor which makes it less likely to be damaged in use or in transit.

Preferably, the sensor strip comprises a frame, preferably wherein the frame is arranged substantially about the outside of the sensor strip, preferably wherein the frame is removable. A frame can provide structural rigidity to the sensor strip. It is advantageous to be around the outside so as to leave the bulk of the sensor free for use. It is a further advantage if the frame is removable, as it can then be reused for subsequent sensor strips.

Preferably, the flexible substrate comprises a polymeric film. Preferably the flexible substrate comprises polyimide, polyethylene, polypropylene, polyethylene terephthalate, polyvinyl chloride, polyamide, polystyrene, polycarbonate, polylactide, cellulose, starch, paper, wax-coated paper, plastic-coated paper, laminated plastic films, or any combination of two or more thereof, preferably polyimide and/ or polyethylene, preferably polyimide, preferably poly (4,4'-oxydiphenylene-pyromellitimide) (Kapton^{®}). Such materials have the advantage of minimal deformation under tension during printing which helps the sensor strip be consistently made. Furthermore, such materials are lightweight and have sufficient tensile strength.

Preferably, the flexible substrate has a thickness in the range of about 10 µm to about 500 µm, preferably about 20 µm to about 250 µm, preferably about 20 µm to about 150 µm. Such thicknesses allow the substrate to be easily handled, whilst minimising the weight of material used. In a further embodiment, the substrate may be thicker than about 1 mm, such as about 1 mm to about 5 mm.

Preferably, the sensor strip comprises about 3 to about 20 semiconductor polymer sensors, preferably about 4 to about 15 semiconductor polymer sensors, preferably about 5 to about 10 semiconductor polymer sensors, preferably about 6 to about 8 semiconductor polymer sensors. These are appropriate numbers of semiconductor polymer sensors to balance the sensitivity of the system with the desire to minimise the materials used in each sensor.

Preferably, the length of the sensor strip is in the range of about 1 cm, to about 12 cm, preferably in the range of about 3 cm to about 10 cm, preferably in the range of about 4 cm to about 9 cm. Where multiple sensor strips are connected together, the length means the length of a repeating unit.

Preferably, the width of the sensor strip is in the range of about 0.5 cm to about 8 cm, preferably in the range of about 2 cm to about 5 cm.

Such lengths and widths are suitably sized for the number of polymer semiconductor sensors.

Preferably the sensor strip is substantially rectangular. This allows for efficient manufacturing with limited waste of substrate material.

Preferably, a plurality of sensors is provided on a roll. Preferably, the first surface of the flexible substrate is the inner layer. Further, the sensors are protected by the subsequent layers of sensors. This is efficient for storage and handling.

Preferably, each sensor is separable from the other sensors. This allows one sensor to be removed at a time. Preferably the sensors are separable by a perforated area or a tear strip. Alternatively, the sensors are separated by cutting, such as with a knife or a pair of scissors. Preferably the sensors are arranged in a single line on the roll. This makes them easier to dispense.

Preferably a plurality of sensors is provided on a cassette. This allows sensor strips to be moved into position in fluid communication with the sampling chamber when needed. The cassette can then move the next sensor strip into position when needed. This provides an easy way to carry and position sensor strips.

Preferably at least the first surface of the sensor strip is provided with a protective layer, preferably the protective layer is a peel off layer, or a wrapper. This protects the sensors from the atmosphere until use.

Preferably, the sensor is a single use sensor. This give more accurate results as the detection is carried out from the same base point. In particular, if a VOC irreversibly binds to a reusable sensor then the following results will be affected by the irreversibly bound VOC. In the present invention, however this is not an issue as a new sensor strip is used each time.

Preferably the system comprises a plurality of sensor strips. This allows multiple uses of the system.

Preferably the system comprises a frame for the sensor strip. This allows the sensor strip to be more easily handled.

Preferably the apparatus further comprises a temperature sensor and/or a humidity sensor. It is advantageous to know the temperature of the soil sample and/or the humidity as these affect the pattern matching approach in the evaluation of the results using our software.

Preferably the system further comprises a trowel, spade or spatula. This allows easy transfer of a soil sample into the sampling chamber.

Preferably the system further comprises a brush. This allows the sampling chamber to be cleaned between samples.

Preferably the system further comprises a bag. This can be used to store and carry the system.

Preferably, the soil volatile organic compound (VOC) sensor strip is provided within a sensor strip storage device; preferably, wherein the sensor strip storage device is substantially sealed; and/or wherein the sensor strip storage device is a cassette.

It is to be understood that the soil VOC sensor strip is suitable for detecting VOCs in soil and/or detecting gases emitted from soil.

The present invention relates to a method of analysing soil comprising:
a) providing an apparatus, wherein the apparatus comprises a sampling chamber, a sensor strip aperture in the sampling chamber, a power source and an electrical resistance detector; a display and/or data storage;
b) providing a soil volatile organic compound (VOC) sensor strip,
   wherein the sensor strip comprises a flexible substrate with a first surface and an array of semiconductor polymer sensors arranged on the first surface,
   wherein each of the semiconductor polymer sensors comprises a pair of electrodes, wherein the pair of electrodes comprises a first electrode and a second electrode, wherein a semiconductor polymer is disposed between the first electrode and the second electrode;
c) providing a soil sample,
d) positioning the sensor strip over the sensor strip aperture, wherein one or each of the semiconductor polymer sensors are in fluid communication with the sampling chamber, wherein each pair of electrodes is electrically connected to the power source and the electrical resistance detector,
e) positioning the soil sample in the sampling chamber,
f) actuating the power source to supply electricity to the sensor strip, and
g) detecting the electrical resistance of one or each of the semiconductor polymer sensor using the electrical resistance detector to give an output;
h) transmitting output from the electrical resistance detector to the display and/or the data storage; sending the output for comparison with a known dataset and displaying a comparison output on the display and/or storing on the data storage.

This provides a straightforward method of analysing soil health . This allows the output to be viewed or stored.

Preferably the sensor strip is positioned before the sampling chamber containing the soil sample is moved to the closed position.

Preferably, the method further comprises
i) comparing the output of each semiconductor polymer sensor with a reference dataset, preferably using a machine learning algorithm to look for patterns in the output.

This allows the VOC fingerprint to be compared with previous data and to advice on differences.

Preferably, the method further comprises:
j) providing soil care advice based on step i), preferably wherein the soil care advice comprises adjusting pH level, water content, nitrate content, microbial biomass or fungi content, or any combination of two or more thereof.

Preferably microbial biomass may be increased by providing an organic fertilizer, preferably manure or compost.

Preferably the advice is provided on a display. Preferably the advice is provided within 1 hour of the actuation of the power source, preferably between about 2 minutes and about 30 minutes, preferably between about 4 minutes and about 10 minutes, preferably between about 5 minutes and about 8 minutes.

This allows the user to be given advice on how to improve the condition of the soil. The comparison of the fingerprint generated by each semiconductor polymer sensor with the reference dataset suggests advice that can be implemented by the user. The real time advice is an advantage of the invention.

Preferably the reference dataset comprises a series of outputs from the semiconductor polymer sensors, combined with other information about the soil, such as the water content, humidity, temperature, crops grown, stage of crops, yield of crops or microbial diversity or any combination of two or more thereof.

Preferably step d) further comprises securing the sensor strip, preferably using a sensor strip securing device. Preferably, the sensor strip securing device is attached to the housing. Preferably, the sensor strip securing device is pivotably attached to the housing. Preferably wherein the sensor strip securing device is a lever movable from an open position to a closed position. It is advantageous to be able to hold the sensor strip in position. Typically, the system will be used outside, such as in a field where it will be exposed to the elements, such as to the wind. It is advantageous for the sensor strip to be held in position such that it does not substantially move or blow away. The aim of this is to ensure that each of the first electrode and the second electrode are connected to the appropriate electrical connections within the apparatus.

Preferably, step c) further comprises heating the soil sample.

It is an advantage to heat the soil to release VOCs from the soil sample and/or release gases adsorbed by the soil particles.

Preferably, step d) further comprises inserting a sensor strip storage device into the apparatus; preferably, wherein the sensor strip storage device is a cassette.

Preferably, step d) further comprises positioning the sensor strip over the sensor strip aperture by automatic replacement and/or positioning of the sensor strip in fluid communication with the sampling chamber; preferably, wherein automatic replacement is by moving the sensor strip between at least two spools and across the sampling chamber.

Preferably step f) comprises applying about 1 to about 10 volts, preferably about 3 to about 8 volts. It is an advantage that a low voltage can be applied.

Preferably step f) comprises applying direct current (DC). It is advantageous to only require direct current. This simplifies the equipment needed to provide the electricity to the sensor strip. It is an advantage of the invention that DC current can be used.

Alternatively, step f) comprises applying alternating current (AC).

Preferably the method is carried out above about 10 °C. Whilst the method can be carried out below this temperature, the VOC levels are likely to be lower.

It is a further advantage of the invention that the soil can be analysed when it has any water content. It is therefore not necessary to wait for any particular weather conditions before carrying out the analysis. It is preferable that the volumetric water content of the soil is about 1% to about 50%. The volumetric water content may be calculated from the volume of the water divided by the volume of the soil (including any air and water present). Water may be added to the soil sample if the volumetric water content of the soil sample is less than about 1. The present invention relates to a method of improving soil conditions, comprising carrying out the method of analysing soil described herein in steps a) to i), and acting on the soil care advice. This aids the user to treat the soil such that they can increase the yield of crops. This provides the user with real time advice on how to improve soil health.

### DETAILED DESCRIPTION OF THE DRAWINGS

Example embodiments of the present invention will now be described with reference to the accompanying figures.

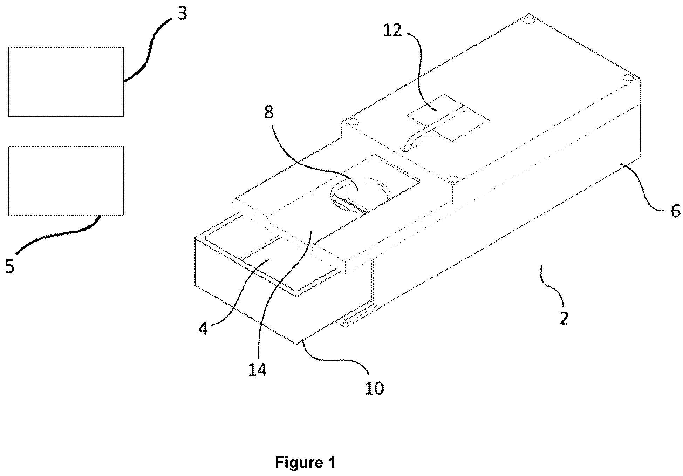

Figure 1 shows an apparatus 2 of the invention. The apparatus 2 comprises a sampling chamber 4. The sampling chamber 4 is in the form of a drawer 10 and is shown in the open position. The sampling chamber 4 is ready to receive the soil sample. A sensor strip aperture 8 is shown positioned above where the sampling chamber 4 will be when it is in the closed position. A sensor strip securing device 12 is shown as a lever and is in the open position. A sensor strip position device 14 is shown as a recess for receiving the sensor strip 20 (not shown). The recess is sized and shaped to receive the sensor strip 20. A housing 6 is shown to contain a power supply (not shown) and an electrical resistance detector (not shown). A display 5 and/or a data storage 3 are connectable to the electrical resistance detector via Bluetooth, Wi-Fi, a mobile wireless communication system a cable or direct electrical connection.

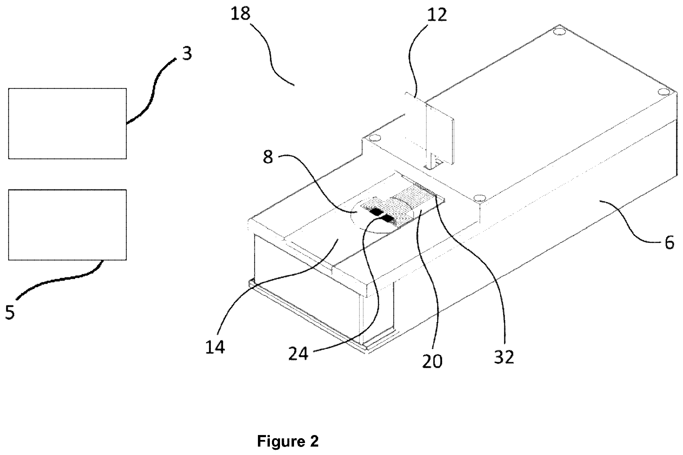

Figure 2 shows a system 18 of the invention including the display 3 and/or a data storage 3. The system is shown with the apparatus of Figure 1 and a sensor strip 20. The drawer 10 is shown in the closed position. The sensor strip 20 is shown positioned in the sensor strip positioning device which is shown as a recess. The sensor strip 20 is positioned with the semiconductor polymer sensors 24 positioned with the first surface of the sensor strip 20 over the sensor strip aperture 8, facing the inside of the sampling chamber 4. The electrical connections 32 are shown connected and are connected to the power supply (not shown) and the electrical resistance detector (not shown). The sensor strip securing device 12 is shown part way between the open position and the closed position. In use, it will be moved to be in contact with the sensor strip 20.

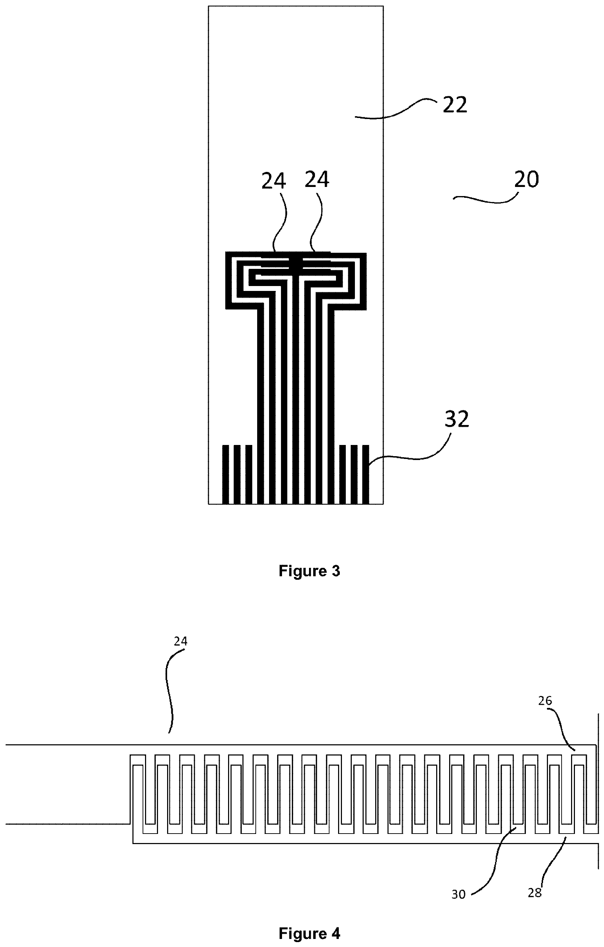

Figure 3 shows a sensor strip 20 of the invention. The sensor strip 20 has a flexible substrate 22 with an array of semiconductor polymer sensors 24. In this example, six sensors are shown, but other numbers are possible as described herein. Each semiconductor polymer sensor 24 is shown connected to the electrical connections 32.

Figure 4 shows a magnified semiconductor polymer sensor 24 of the invention. The semiconductor polymer sensor 24 is shown with a first electrode 26 and a second electrode 28. The first electrode 26 and the second electrode 28 are shown in the form of interdigitated fingers. As shown, the first electrode 26 and the second electrode 28 have substantially perpendicular turns between subsequent fingers and the fingers are substantially straight. In each semiconductor polymer sensor, a semiconductor polymer 30 is disposed between the first electrode 26 and the second electrode 28.

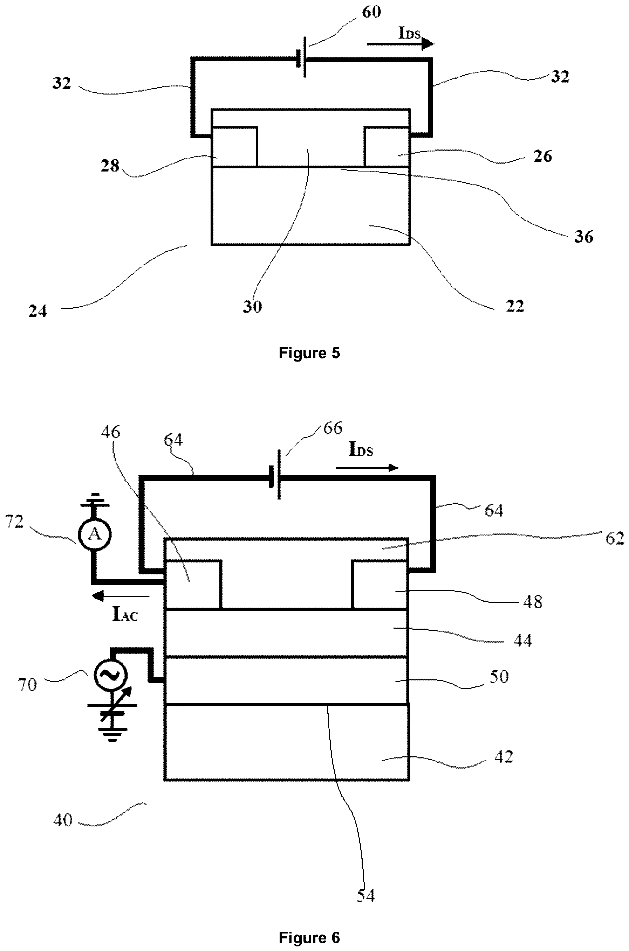

Figure 5 shows a cross section of a semiconductor polymer sensor 24 of the invention. The semiconductor sensor 24 comprises a flexible substrate 22. A semiconductor polymer 30 is arranged on the first surface 36 of the flexible substrate 22 and is disposed between a first electrode 26 and a second electrode 28. Electrical connections 32 connect the first electrode 26 and the second electrode 28 to the power supply 60. As shown the current I_{DS} flows in a clockwise direction from the power supply 60, to the first electrode 26, across the organic semiconductor polymer 30, to the second electrode 28 and back to the power supply 60. The electrical resistance between the first electrode 26 and the second electrode 28 can be measured using an electrical resistance detector (not shown).

Figure 6 shows a cross section of a soil volatile organic compound (VOC) impedance spectroscopy sensor strip 40. The sensor strip 40 is shown as a field effect transistor and has a flexible substrate 42 with a gate electrode 50 on the first surface 54 of the flexible substrate 42. An insulator layer 44 is arranged over the gate electrode 50. A source electrode 46 and a drain electrode 48 are arranged on top of the insulator layer 44. A semiconductor polymer 62 is arranged on the insulator layer 44 and between the source electrode 46 and the drain electrode 48. The insulator layer 44 separates the gate electrode 50 from the source electrode 46, the drain electrode 48 and the semiconductor polymer 62. Electrical connections 64 connect the source electrode 46 and the drain electrode 48 to the power supply 66. As shown the current I_{DS} flows in a clockwise direction from the power supply 66, to the drain electrode 48, across the organic semiconductor polymer 62, to the source electrode 46 and back to the power supply 66. A source of alternating current 70 is supplied to the gate electrode 50. A frequency response analyser 72 measures the frequency of the I_{AC} Further, the electrical resistance between the source electrode 46 and the second electrode 48 can be measured using an electrical resistance detector (not shown). It is necessary to make two measurements, and then to calculate the impedance. This requires more complicated electronics and more computation power than the sensor strip shown in figure 5. Further, the output is more complex than the sensor of figure 5.

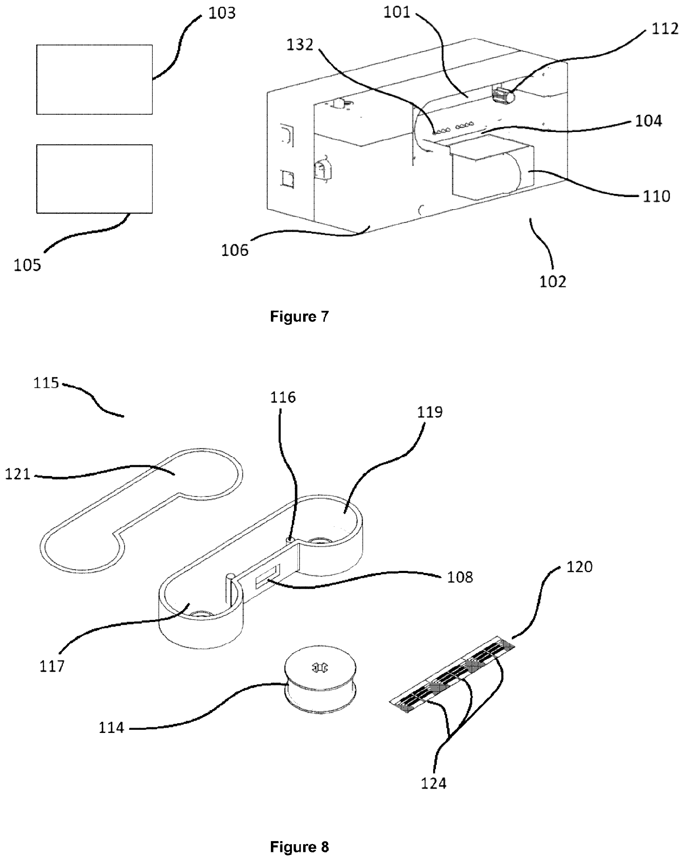

Figure 7 shows a further embodiment of the apparatus of the invention. The apparatus 102 of the further embodiment comprises a sampling chamber 104. The sampling chamber 104 is in the form of a drawer 110 and is shown in the open position in Figure 7. The drawer 11 is moveable into and out of the housing 106. The sampling chamber 104 is shown in Figure 7 ready to receive a soil sample. The apparatus 102 further comprises a housing 106 containing a power supply (not shown), a stepper motor (not shown), and an electrical resistance detector (not shown). A display 105 and/or a data storage 103 are connectable to the electrical resistance detector via Bluetooth, Wi-Fi, a mobile wireless communication system a cable or direct electrical connection. The housing 106 can also additionally comprise a heating element (not shown) to heat the soil in the soil drawer 110 to release VOCs and gases adsorbed by the particles of the soil sample.

Sensor strips 120 are wound onto a cassette spool 114 within a cassette 115, as shown in Figure 8, which, in use, is inserted into a cassette chamber 101. The sensors 124, in the form of a sensor strip 120, are moved into position by a stepper motor (not shown) via the removable engagement of the cassette spool drive shaft 112 with the cassette spool 114. It is understood that alternative movement mechanisms and alternative sensor strip storage means can be used to move the sensors 124 automatically into the required position facing the sampling chamber 104.

In use, to analyse a soil sample, the sensors 124, in the form of a sensor strip 120, make electrical contact with the apparatus 102 for a soil scan, and are then automatically moved on with a new sensor 124 in the sensor strip 120 taking its place. In use, the electrical connectors 132 carry the signal necessary for the connector system to move the sensor strip 120 as required.

Figure 8 shows an exploded view of a cassette 115 for use with the further embodiment of the apparatus of the invention, as shown in Figure 7. The cassette 115 comprises a first cassette spool housing chamber 117, a second cassette spool housing chamber 119, a cassette housing lid 121, a sensor aperture 108 in the cassette 115, a first cassette spool 114, a second cassette spool (not shown), guides 116 for positioning the sensor strip 120, and the sensors 124. The sensor strip 120 is shown in Figure 8 to include a short strip of sensors 124; however, it is understood that in use a long strip 120 of around 10-1000 or more sensors is wound onto the respective cassette spool 114. The sensor strip 124 comprises sensors 124 as described with respect to Figure 3. In a preferred embodiment of the present invention, the width of each sensor 124 is about 1.5cm and the length of each sensor 124 is about 3cm with the length of substrate, i.e. the gap, between each sensor having a length of about 3cm. In alternative embodiments, the sensors 124 are positioned closer together to reduce the length of the sensor strip 120; for example, the length of substrate between each sensor 124 is between about 1cm and about 5cm. In alternative embodiments, the width of each sensor is about 1cm and the length of each sensor is about 2cm. It is understood that the sensor strip 120 of the present invention is carefully configured to balance efficiency of production and achieving the required sensitivity to VOCs and/or gases that are adsorbed. A sensor strip having a reduced surface area will be more efficient for production but will have a reduced sensitivity.

Prior to use, the sensor strip 120 can be packaged in sealed nitrogen-containing packaging to prevent exposure of the sensors 124 to air, oxygen, humidity, or other contaminants.

In use, one or more new, unused sensor strips 120 are wound tightly around a first cassette spool 114, with the last sensor in the strip attached to a second cassette spool (not shown). Thus, the new sensors 124 in the form of a sensor strip 120 are wound from one cassette spool across the sensor aperture 108 and held in a scan position, and subsequently wound tightly onto the other cassette spool as they are automatically moved in use. The sensors 124, as part of the sensor strip 120, pass by the sensor aperture 108 via the guides 116 to allow the sensor to make electrical contact with the apparatus, shown in Figure 7. Tight winding of the sensor strip/s 120 around the respective cassette spools 114 ensures that air and humidity is excluded from each sensor surface to extend the lifetime of the sensors 124 on the sensor strip 120 by protecting them from degradation by atmospheric conditions.

### EXAMPLE

The invention will now be described with reference to the following non-limiting example.

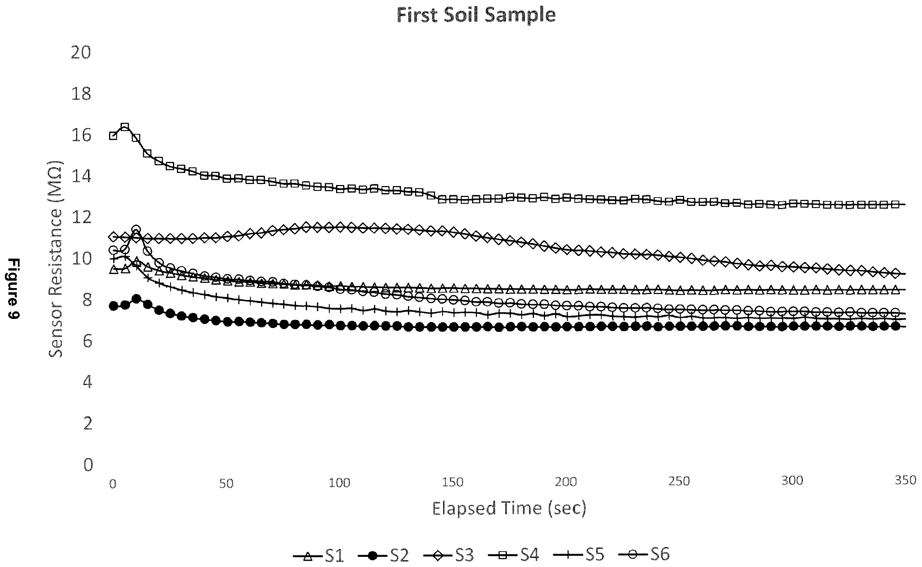

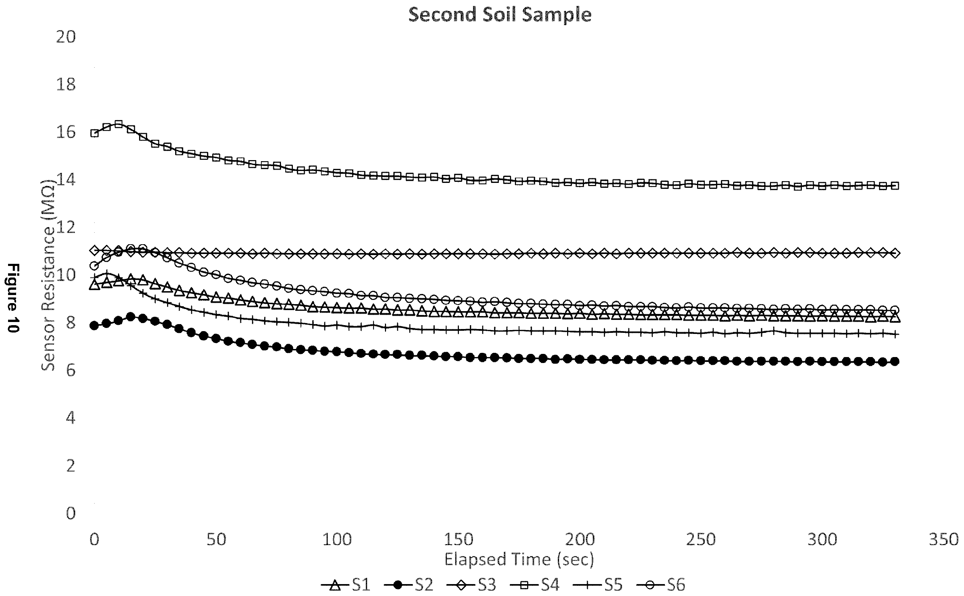

A sensor strip with an array of six semiconductor polymer sensors was used in an apparatus of the invention to measure the output from a first soil sample and a second soil sample. The soil samples had different compositions. The sensor strip was positioned over the sensor strip aperture and the first soil sample was positioned in the sampling chamber. Electricity was applied to the sensor strip and the electrical resistance was measured for each semiconductor polymer sensor over at least 5 minutes. This was repeated with a new sensor strip for the second soil sample. The results are shown in a graph of resistance over time in Figure 9 for the first soil sample and figure 10 for the second soil sample. As shown in figure 9, each semiconductor polymer sensor produced a different pattern compared with the other semiconductor polymer sensors indicating that each semiconductor polymer sensor has a different response to the VOCs from the first soil sample. Analogous results are shown in figure 10. Further each soil sample produced a different plot for a specific semiconductor polymer sensor, that is S1 (sensor 1) in figure 9 shows a different pattern to S1 (sensor 1) in figure 10 and so on for each of the six sensors. By comparing these patterns to a reference dataset, attributes of the soil may be analysed. The present invention using a machine learning algorithm to compare the pattern from each sensor to a known dataset. This allows the user to identify key features of the soil and to be provided with advice on how to improve the quality of the soil.

Within this specification embodiments have been described in a way which enables a clear and concise specification to be written, but it is intended and will be appreciated that embodiments may be variously combined or separated without parting from the invention. For example, it will be appreciated that all preferred features described herein are applicable to all aspects of the invention described herein and vice versa.

Within this specification, the term "about" means plus or minus 20%, more preferably plus or minus 10%, even more preferably plus or minus 5%, most preferably plus or minus 2%.

Within this specification, the term "substantially" means a deviation of plus or minus 20%, more preferably plus or minus 10%, even more preferably plus or minus 5%, most preferably plus or minus 2%.

Within this specification, the term "one or each", preferably means at least one, preferably each.

It should be understood that various changes and modifications to the presently preferred embodiments described herein will be apparent to those skilled in the art. Such changes and modifications can be made without departing from the scope of the present invention and without diminishing its attendant advantages. It is therefore intended that such changes and modifications are covered by the appended claims.

## Claims

1. A system (18) for analysing volatile organic compounds (VOCs) in soil comprising an apparatus (2; 102) and a soil VOC sensor strip (20; 40),
wherein the apparatus (102) comprises a sampling chamber (104) for receiving soil, a sensor strip aperture (8; 108) in the sampling chamber (4; 104) for positioning the sensor strip (20; 40) in fluid communication with the sampling chamber (104), a power source and an electrical resistance detector,
wherein the sensor strip (20; 40) is electrically connectable to the power source (60) and the electrical resistance detector, and
wherein the system further comprises a display (5; 105) and/or data storage (3; 103), wherein the electrical resistance detector is configured to transmit output to the display (5; 105) and/or the data storage (3; 103), and wherein the data storage (3; 103) is configured to send the output for comparison with a known dataset and to store a comparison output, and the display (5; 105) is configured to display the comparison output,
**characterised in that** the sensor strip (20; 40) comprises a flexible substrate (42) with a first surface (54) and an array of semiconductor polymer sensors (24; 124) arranged on the first surface (54), wherein each of the semiconductor polymer sensors (24; 124) comprises a pair of electrodes, wherein the pair of electrodes comprises a first electrode (26; 46) and a second electrode (28; 48), wherein a semiconductor polymer (30; 62) is disposed between the first electrode (26; 46) and the second electrode (28; 48).

2. A system according to any preceding claim, wherein the apparatus (2; 102) further comprises:
i. a housing (106), preferably wherein the housing (106) comprises the sampling chamber (104), preferably wherein the housing (106) further comprises the electrical resistance detector and/or the power source, preferably wherein the housing (106) further comprises the electrical resistance detector and the power source; and/or
ii. wherein the display (105) and/or data storage (103) is connectable to the electrical resistance detector via Bluetooth, Wi-Fi, a mobile wireless communication system a cable or direct electrical connection, preferably via Bluetooth, Wi-Fi or a mobile wireless communication system, preferably via Bluetooth; and/or
wherein the display (105) and/or data storage (103) is a personal electronic device (PED), preferably the PED is a smart phone, a tablet, a mobile phone, a laptop or a notebook, preferably a smart phone; and/or
iii. wherein the apparatus (102) further comprises a sensor strip securing device, preferably wherein the sensor strip securing device is attached to the housing (106), preferably wherein the sensor strip securing device is pivotably attached to the housing (106), preferably wherein the sensor strip securing device is a lever movable from an open position to a closed position; and/or
iv. wherein the apparatus (102) comprises a heater for heating the sampling chamber (104).

3. A system according to any preceding claim, wherein the apparatus (102) further comprises a sensor strip storage device; preferably wherein the sensor strip storage device is substantially sealed; and/or wherein the sensor strip storage device is a cassette (115); and/or
wherein the apparatus (102) further comprises at least one movement mechanism for automatic replacement and/or positioning of the sensor strip (20; 40) in fluid communication with the sampling chamber (104); preferably, wherein the movement mechanism comprises at least two spools (114) for moving the sensor strip (20; 40) therebetween and across the sampling chamber (104); optionally, wherein the movement mechanism comprises a stepper motor and/or a sensor strip positioning device.

4. A system according to any preceding claim, wherein the sampling chamber (104) has an open position and a closed position, preferably wherein the sampling chamber (104) is a drawer and the drawer is movable from the open position to the closed position; and/or
wherein the apparatus (102) further comprises a switch, wherein actuating the switch turns on the power source, preferably wherein the switch is actuated when the sampling chamber (104) is in the closed position; and/or
wherein the power source is turned off after a predetermined period of time.

5. A system according to any preceding claim, wherein the apparatus (102) is a handheld apparatus.

6. A system according to any preceding claim, wherein the sampling chamber (104) has a maximum capacity in a range of about 10 cm³ to about 300 cm³; and/or
wherein the distance between the level of the soil when the sampling chamber (104) is filled to maximum capacity, and the sensor strip (20; 40), is in the range of about 0.5 cm to 5 cm, preferably about 1 cm to about 3 cm; and/or
wherein the headroom when the sampling chamber (104) is filled to maximum capacity and when the sampling chamber (104) is in the closed position has a volume in the range of about 3 cm³ to about 50 cm³.

7. A system according to any preceding claim, wherein one or each of the pair of electrodes are in the form of interdigitated fingers or concentric spirals; and/or
wherein one or each of the first electrode (26; 46) and/or one or each of the second electrode (28; 48) comprises a metal, a metal oxide, an electrically conductive polymer, graphene or carbon, preferably, silver, gold, copper, zinc, carbon, graphene nanoplatelets, indium tin oxide, poly(3,4-ethylenedioxythiophene) polystyrene sulfonate (PEDOT:PSS), 3,4-ethylenedioxythiophene or a derivative thereof or poly(3,4-ethylenedioxythiophene or a derivative thereof or any combination of two or more thereof; and/or
wherein the distance between the first electrode (26; 46) and the second electrode (28; 48) of one or each of the pair of electrodes is in the range of about 0.1 µm to about 40 µm.

8. A system according to any preceding claim, wherein one or each of the first electrode (26; 46) and/or one or each of the second electrode (28; 48) are printed, preferably flexography printed, gravure printed, offset printed, screen printed or digital printed, and/or
wherein one or each of the semiconductor polymers (30; 62) are printed.

9. A system according to any preceding claim, wherein the length of one or each of the first electrode (26; 46) and/or one or each of the second electrode (28; 48) is in the range of about 1 cm to about 50 cm; and/or
wherein the width and thickness of one or each of the first electrode (26; 46) and/or one or each of the second electrode (28; 48) is each independently in the range of about 3 nm to about 1.5 µm; and/or
wherein one or each of the semiconductor polymers (30; 62) is an organic semiconductor polymer; and/or
wherein one or each of the semiconductor polymers (30; 62) has a molecular weight of about 10,000 to about 500,000.

10. A system according to any preceding claim, wherein at least two of the semiconductor polymers (30; 62) comprises a different semiconductor polymer; and/or
wherein one or each of the semiconductor polymers (30; 62) has a thickness of about 1 nm to about 100 µm.

11. A system according to any preceding claim, wherein:
v) the sensor strip (20; 40) comprises a backing layer on a second surface of the flexible substrate (42), wherein the second surface opposes the first surface (54), preferably wherein the backing layer comprises paper, card, or a polymeric film; and/or
vi) wherein the sensor strip (20; 40) comprises a frame wherein the frame is arranged substantially about the outside of the sensor strip (20; 40), preferably wherein the frame is removable; and/or
vii) wherein the flexible substrate (42) comprises polyimide, polyethylene, polypropylene, polyethylene terephthalate, polyvinyl chloride, polyamide, polystyrene, polycarbonate, polylactide, cellulose, starch, paper, wax-coated paper, plastic-coated paper, laminated plastic films, or any combination of two or more thereof.

12. A system according to any preceding claim, wherein the flexible substrate (42) has a thickness in the range of about 10 µm to about 1 mm; and/or
wherein the sensor strip (20; 40) comprises about 3 to about 20 semiconductor polymer sensors (24; 124); and/or
wherein the length of the sensor strip (20; 40) is in the range of about 1 cm to about 12 cm; and/or
wherein the width of the sensor strip (20; 40) is in the range of about 0.5 cm to about 8 cm.

13. A method of analysing soil comprising:
a) providing an apparatus (102), wherein the apparatus (102) comprises a sampling chamber (104), a sensor strip aperture (108) in the sampling chamber (104), a power source and an electrical resistance detector; a display (105) and/or data storage (103);
b) providing a soil volatile organic compound (VOC) sensor strip (20; 40),
wherein the sensor strip (20; 40) comprises a flexible substrate (42) with a first surface (54) and an array of semiconductor polymer sensors (24; 124) arranged on the first surface (54), wherein each of the semiconductor polymer sensors (24; 124) comprises a pair of electrodes, wherein the pair of electrodes comprises a first electrode (26; 46) and a second electrode (28; 48), wherein a semiconductor polymer (30; 62) is disposed between the first electrode (26; 46) and the second electrode (28; 48);
c) providing a soil sample,
d) positioning the sensor strip (20; 40) over the sensor strip aperture (108), wherein one or each of the semiconductor polymer sensors (24; 124) are in fluid communication with the sampling chamber (104), wherein each pair of electrodes is electrically connected to the power source and the electrical resistance detector,
e) positioning the soil sample in the sampling chamber (104),
f) actuating the power source to supply electricity to the sensor strip (20; 40), and
g) detecting the electrical resistance of one or each of the semiconductor polymer sensor using the electrical resistance detector to give an output;
h) transmitting output from the electrical resistance detector to the display (105) and/or the data storage (103); sending the output for comparison with a known dataset and displaying a comparison output on the display (105) and/or storing on the data storage (103).

14. A method according to claim 13, further comprising:
i) using a machine learning algorithm to look for patterns in the output; optionally further comprising:
j) providing soil care advice based on step i), preferably wherein the soil care advice comprises adjusting soil organic matter content, pH level, water content, nitrate content, microbial biomass or fungi content, or any combination of two or more thereof.

## Patentansprüche

1. System (18) zur Analyse von flüchtigen organischen Verbindungen (VOCs) im Boden, das eine Einrichtung (2; 102) und einen Boden-VOC-Sensorstreifen (20; 40) umfasst,
wobei die Einrichtung (102) eine Probenkammer (104) zum Aufnehmen von Boden, eine Sensorstreifenöffnung (8; 108) in der Probenkammer (4; 104) zum Positionieren des Sensorstreifens (20; 40) in Fluidkommunikation mit der Probenkammer (104), eine Stromquelle und einen elektrischen Widerstandsdetektor umfasst,
wobei der Sensorstreifen (20; 40) elektrisch mit der Stromquelle (60) und dem elektrischen Widerstandsdetektor verbindbar ist, und
wobei das System ferner eine Anzeige (5; 105) und/oder einen Datenspeicher (3; 103) umfasst, wobei der elektrische Widerstandsdetektor dazu konfiguriert ist, eine Ausgabe an die Anzeige (5; 105) und/oder den Datenspeicher (3; 103) zu übertragen, und wobei der Datenspeicher (3; 103) dazu konfiguriert ist, die Ausgabe zum Vergleich mit einem bekannten Datensatz zu übertragen und eine Vergleichsausgabe zu speichern, und die Anzeige (5; 105) dazu konfiguriert ist, die Vergleichsausgabe anzuzeigen,
**dadurch gekennzeichnet, dass** der Sensorstreifen (20; 40) ein flexibles Substrat (42) mit einer ersten Oberfläche (54) und einer Anordnung von Halbleiterpolymersensoren (24; 124) umfasst, die auf der ersten Oberfläche (54) angeordnet sind, wobei jeder der Halbleiterpolymersensoren (24; 124) ein Elektrodenpaar umfasst, wobei das Elektrodenpaar eine erste Elektrode (26; 46) und eine zweite Elektrode (28; 48) umfasst, wobei ein Halbleiterpolymer (30; 62) zwischen der ersten Elektrode (26; 46) und der zweiten Elektrode (28; 48) angeordnet ist.

2. System nach einem vorhergehenden Anspruch, wobei die Einrichtung (2; 102) ferner Folgendes umfasst:
i. ein Gehäuse (106), wobei das Gehäuse (106) vorzugsweise die Probenkammer (104) umfasst, wobei das Gehäuse (106) vorzugsweise ferner den elektrischen Widerstandsdetektor und/oder die Stromquelle umfasst, wobei das Gehäuse (106) vorzugsweise ferner den elektrischen Widerstandsdetektor und die Stromquelle umfasst; und/oder
ii. wobei die Anzeige (105) und/oder der Datenspeicher (103) über Folgendes mit dem elektrischen Widerstandsdetektor verbindbar sind/ist, Bluetooth, WLAN, ein mobiles drahtloses Kommunikationssystem, ein Kabel oder eine direkte elektrische Verbindung, vorzugsweise über Bluetooth, WLAN oder ein mobiles drahtloses Kommunikationssystem, vorzugsweise über Bluetooth; und/oder
wobei es sich bei der Anzeige (105) und/oder dem Datenspeicher (103) um eine persönliche elektronische Vorrichtung (PED) handelt, es sich bei der PED vorzugsweise um ein Smartphone, ein Tablet, ein Mobiltelefon, einen Laptop oder ein Notebook handelt, vorzugsweise um ein Smartphone; und/oder
iii. wobei die Einrichtung (102) ferner eine Sensorstreifensicherungsvorrichtung umfasst, wobei die Sensorstreifensicherungsvorrichtung vorzugsweise an dem Gehäuse (106) angebracht ist, wobei die Sensorstreifensicherungsvorrichtung vorzugsweise schwenkbar an dem Gehäuse (106) angebracht ist, wobei die Sensorstreifensicherungsvorrichtung vorzugsweise ein Hebel ist, der aus einer offenen Position in eine geschlossene Position bewegbar ist; und/oder
iv. wobei die Einrichtung (102) eine Heizung zum Heizen der Probenkammer (104) umfasst.

3. System nach einem der vorhergehenden Ansprüche, wobei die Einrichtung (102) ferner eine Sensorstreifenspeichervorrichtung umfasst; wobei die Sensorstreifenspeichervorrichtung vorzugsweise im Wesentlichen versiegelt ist; und/oder wobei die Sensorstreifenspeichervorrichtung eine Kassette (115) ist; und/oder
wobei die Einrichtung (102) ferner mindestens einen Bewegungsmechanismus zum automatischen Austauschen und/oder Positionieren des Sensorstreifens (20; 40) in Fluidkommunikation mit der Probenkammer (104) umfasst; wobei der Bewegungsmechanismus vorzugsweise mindestens zwei Spulen (114) zum Bewegen des Sensorstreifens (20; 40) dazwischen und über die Probenkammer (104) umfasst; wobei der Bewegungsmechanismus optional einen Schrittmotor und/oder eine Sensorstreifenpositionierungsvorrichtung umfasst.

4. System nach einem der vorhergehenden Ansprüche, wobei die Probenkammer (104) eine offene und eine geschlossene Position aufweist, wobei die Probenkammer (104) vorzugsweise eine Schublade ist und die Schublade aus der offenen in die geschlossene Position bewegbar ist; und/oder
wobei die Einrichtung (102) ferner einen Schalter umfasst, wobei durch Betätigen des Schalters die Stromquelle eingeschaltet wird, wobei der Schalter vorzugsweise betätigt wird, wenn sich die Probenkammer (104) in der geschlossenen Position befindet; und/oder
wobei die Stromquelle nach einem vorbestimmten Zeitraum ausgeschaltet wird.

5. System nach einem der vorhergehenden Ansprüche, wobei es sich bei der Einrichtung (102) um eine Handeinrichtung handelt.

6. System nach einem der vorhergehenden Ansprüche, wobei die Probenkammer (104) eine maximale Kapazität in einem Bereich von etwa 10 cm³ bis etwa 300 cm³ aufweist; und/oder
wobei die Entfernung zwischen dem Bodenniveau, wenn die Probenkammer (104) bis zur maximalen Kapazität gefüllt ist, und dem Sensorstreifen (20; 40) im Bereich von etwa 0,5 cm bis 5 cm, vorzugsweise etwa 1 cm bis etwa 3 cm, liegt; und/oder
wobei die Kopffreiheit, wenn die Probenkammer (104) bis zur maximalen Kapazität gefüllt ist und wenn sich die Probenkammer (104) in der geschlossenen Position befindet, ein Volumen im Bereich von etwa 3 cm³ bis etwa 50 cm³ aufweist.

7. System nach einem der vorhergehenden Ansprüche, wobei eine oder jede Elektrode des Elektrodenpaars die Form von ineinandergreifenden Fingern oder konzentrischen Spiralen hat; und/oder
wobei eine oder jede der ersten Elektrode (26; 46) und/oder eine oder jede der zweiten Elektrode (28; 48) Folgendes umfassen/umfasst, ein Metall, ein Metalloxid, ein elektrisch leitfähiges Polymer, Graphen oder Kohlenstoff, vorzugsweise Silber, Gold, Kupfer, Zink, Kohlenstoff, Graphen-Nanoplättchen, Indiumzinnoxid, Poly(3,4-ethylendioxythiophen)-polystyrolsulfonat (PEDOT:PSS), 3,4-Ethylendioxythiophen oder ein Derivat davon oder Poly(3,4-ethylendioxythiophen) oder ein Derivat davon oder eine beliebige Kombination aus zwei oder mehreren davon; und/oder
wobei die Entfernung zwischen der ersten Elektrode (26; 46) und der zweiten Elektrode (28; 48) einer oder jeder Elektrode des Elektrodenpaars im Bereich von etwa 0,1 µm bis etwa 40 µm liegt.

8. System nach einem der vorhergehenden Ansprüche, wobei eine oder jede der ersten Elektrode (26; 46) und/oder eine oder jede der zweiten Elektrode (28; 48) gedruckt sind/ist, vorzugsweise im Flexodruckverfahren, im Tiefdruckverfahren, im Offsetdruckverfahren, im Siebdruckverfahren oder im Digitaldruckverfahren, und/oder
wobei eines oder jedes der Halbleiterpolymere (30; 62) gedruckt ist.

9. System nach einem der vorhergehenden Ansprüche, wobei die Länge einer oder jeder der ersten Elektrode (26; 46) und/oder einer oder jeder der zweiten Elektrode (28; 48) im Bereich von etwa 1 cm bis etwa 50 cm liegt; und/oder
wobei die Breite und Dicke einer oder jeder der ersten Elektrode (26; 46) und/oder einer oder jeder der zweiten Elektrode (28; 48) jeweils unabhängig im Bereich von etwa 3 nm bis etwa 1,5 µm liegt; und/oder
wobei eines oder jedes der Halbleiterpolymere (30; 62) ein organisches Halbleiterpolymer ist; und/oder
wobei eines oder jedes der Halbleiterpolymere (30; 62) ein Molekulargewicht von etwa 10.000 bis etwa 500.000 aufweist.

10. System nach einem der vorhergehenden Ansprüche, wobei mindestens zwei der Halbleiterpolymere (30; 62) ein unterschiedliches Halbleiterpolymer umfassen; und/oder
wobei eines oder jedes der Halbleiterpolymere (30; 62) eine Dicke von etwa 1 nm bis etwa 100 µm aufweist.

11. System nach einem vorhergehenden Anspruch, wobei:
v) der Sensorstreifen (20; 40) eine Trägerschicht auf einer zweiten Oberfläche des flexiblen Substrats (42) umfasst, wobei die zweite Oberfläche der ersten Oberfläche (54) gegenüberliegt, wobei die Trägerschicht vorzugsweise Papier, Karton oder eine Polymerfolie umfasst; und/oder
vi) wobei der Sensorstreifen (20; 40) einen Rahmen umfasst, wobei der Rahmen im Wesentlichen um die Außenseite des Sensorstreifens (20; 40) herum angeordnet ist, wobei der Rahmen vorzugsweise abnehmbar ist; und/oder
vii) wobei das flexible Substrat (42) Folgendes umfasst, Polyimid, Polyethylen, Polypropylen, Polyethylenterephthalat, Polyvinylchlorid, Polyamid, Polystyrol, Polycarbonat, Polylactid, Zellulose, Stärke, Papier, wachsbeschichtetes Papier, kunststoffbeschichtetes Papier, laminierte Kunststofffolien oder eine beliebige Kombination aus zwei oder mehreren davon.

12. System nach einem der vorhergehenden Ansprüche, wobei das flexible Substrat (42) eine Dicke im Bereich von etwa 10 µm bis etwa 1 mm aufweist; und/oder
wobei der Sensorstreifen (20; 40) etwa 3 bis etwa 20 Halbleiterpolymersensoren (24; 124) umfasst; und/oder
wobei die Länge des Sensorstreifens (20; 40) im Bereich von etwa 1 cm bis etwa 12 cm liegt; und/oder
wobei die Breite des Sensorstreifens (20; 40) im Bereich von etwa 0,5 cm bis etwa 8 cm liegt.

13. Verfahren zur Analyse von Boden, umfassend:
a) Bereitstellen einer Einrichtung (102), wobei die Einrichtung (102) eine Probenkammer (104), eine Sensorstreifenöffnung (108) in der Probenkammer (104), eine Stromquelle und einen elektrischen Widerstandsdetektor umfasst; einer Anzeige (105) und/oder eines Datenspeichers (103);
b) Bereitstellen eines Sensorstreifens (20; 40) für flüchtige organische Verbindungen (VOC) im Boden,
wobei der Sensorstreifen (20; 40) ein flexibles Substrat (42) mit einer ersten Oberfläche (54) und eine Anordnung von Halbleiterpolymersensoren (24; 124) umfasst, die auf der ersten Oberfläche (54) angeordnet sind, wobei jeder der Halbleiterpolymersensoren (24; 124) ein Elektrodenpaar umfasst, wobei das Elektrodenpaar eine erste Elektrode (26; 46) und eine zweite Elektrode (28; 48) umfasst, wobei ein Halbleiterpolymer (30; 62) zwischen der ersten Elektrode (26; 46) und der zweiten Elektrode (28; 48) angeordnet ist;
c) Bereitstellung einer Bodenprobe,
d) Positionieren des Sensorstreifens (20; 40) über der Sensorstreifenöffnung (108), wobei einer oder jeder der Halbleiterpolymersensoren (24; 124) in Fluidkommunikation mit der Probenkammer (104) steht, wobei jedes Elektrodenpaar elektrisch mit der Stromquelle und dem elektrischen Widerstandsdetektor verbunden ist,
e) Positionieren der Bodenprobe in der Probenkammer (104),
f) Betätigen der Stromquelle, um den Sensorstreifen (20; 40) mit Elektrizität zu versorgen, und
g) Detektieren des elektrischen Widerstands eines oder jedes Halbleiterpolymersensors unter Verwendung des elektrischen Widerstandsdetektors, um eine Ausgabe abzugeben;
h) Übertragen der Ausgabe von dem elektrischen Widerstandsdetektor an die Anzeige (105) und/oder den Datenspeicher (103); Senden der Ausgabe zum Vergleich mit einem bekannten Datensatz und Anzeigen einer Vergleichsausgabe auf der Anzeige (105) und/oder Speichern auf dem Datenspeicher (103).

14. Verfahren nach Anspruch 13, ferner umfassend:
i) Verwenden eines Algorithmus des maschinellen Lernens zum Suchen nach Mustern in der Ausgabe; optional ferner umfassend:
j) Bereitstellung von Bodenpflegeratschlägen auf Grundlage von Schritt i), wobei die Bodenpflegeratschläge vorzugsweise Anpassen des Gehalts an organischer Substanz im Boden, des pH-Werts, des Wassergehalts, des Nitratgehalts, der mikrobiellen Biomasse oder des Pilzgehalts oder einer beliebigen Kombination aus zwei oder mehreren davon umfassen.

## Revendications

1. Système (18) permettant l'analyse de composés organiques volatils (COV) dans le sol comprenant un appareil (2 ; 102) et une bande de capteur de COV dans le sol (20 ; 40),
dans lequel l'appareil (102) comprend une chambre d'échantillonnage (104) pour recevoir le sol, une ouverture de bande de capteur (8 ; 108) dans la chambre d'échantillonnage (4 ; 104) pour positionner la bande de capteur (20 ; 40) en communication fluidique avec la chambre d'échantillonnage (104), une source d'alimentation et un détecteur de résistance électrique,
dans lequel la bande de capteur (20 ; 40) peut être connectée électriquement à la source d'alimentation (60) et au détecteur de résistance électrique, et
dans lequel le système comprend en outre un affichage (5 ; 105) et/ou un stockage de données (3 ; 103), dans lequel le détecteur de résistance électrique est configuré pour transmettre une sortie à l'affichage (5 ; 105) et/ou au stockage de données (3 ; 103), et dans lequel le stockage de données (3 ; 103) est configuré pour envoyer la sortie pour comparaison à un ensemble de données connu et pour stocker une sortie de comparaison, et l'affichage (5 ; 105) est configuré pour afficher la sortie de comparaison,
**caractérisé en ce que** la bande de capteur (20 ; 40) comprend un substrat flexible (42) avec une première surface (54) et un réseau de capteurs polymères semi-conducteurs (24 ; 124) agencés sur la première surface (54), dans lequel chacun des capteurs polymères semi-conducteurs (24 ; 124) comprend une paire d'électrodes, dans lequel la paire d'électrodes comprend une première électrode (26 ; 46) et une seconde électrode (28 ; 48), dans lequel un polymère semi-conducteur (30 ; 62) est disposé entre la première électrode (26 ; 46) et la seconde électrode (28 ; 48).

2. Système selon l'une quelconque des revendications précédentes, dans lequel l'appareil (2 ; 102) comprend en outre :
i. un boîtier (106), de préférence dans lequel le boîtier (106) comprend la chambre d'échantillonnage (104), de préférence dans lequel le boîtier (106) comprend en outre le détecteur de résistance électrique et/ou la source d'alimentation, de préférence dans lequel le boîtier (106) comprend en outre le détecteur de résistance électrique et la source d'alimentation ; et/ou
ii. dans lequel l'affichage (105) et/ou le stockage de données (103) peuvent être connectés au détecteur de résistance électrique via Bluetooth, Wi-Fi, un système de communication sans fil mobile, une connexion électrique câblée ou directe, de préférence via Bluetooth, Wi-Fi ou un système de communication sans fil mobile, de préférence via Bluetooth ; et/ou
dans lequel l'affichage (105) et/ou le stockage de données (103) est un dispositif électronique personnel (PED), de préférence le PED est un téléphone intelligent, une tablette, un téléphone mobile, un ordinateur portable ou un ordinateur notebook, de préférence un téléphone intelligent ; et/ou
iii. dans lequel l'appareil (102) comprend en outre un dispositif de fixation de bande de capteur, de préférence dans lequel le dispositif de fixation de bande de capteur est attaché au boîtier (106), de préférence dans lequel le dispositif de fixation de bande de capteur est attaché de manière pivotante au boîtier (106), de préférence dans lequel le dispositif de fixation de bande de capteur est un levier mobile d'une position ouverte à une position fermée ; et/ou
iv. dans lequel l'appareil (102) comprend un élément chauffant pour chauffer la chambre d'échantillonnage (104).

3. Système selon l'une quelconque des revendications précédentes, dans lequel l'appareil (102) comprend en outre un dispositif de stockage de bande de capteur ; de préférence dans lequel le dispositif de stockage de bande de capteur est sensiblement étanche ; et/ou dans lequel le dispositif de stockage de bande de capteur est une cassette (115) ; et/ou
dans lequel l'appareil (102) comprend en outre au moins un mécanisme de mouvement pour le remplacement et/ou le positionnement automatique de la bande de capteur (20 ; 40) en communication fluidique avec la chambre d'échantillonnage (104) ; de préférence, dans lequel le mécanisme de mouvement comprend au moins deux bobines (114) pour déplacer la bande de capteur (20 ; 40) entre elles et à travers la chambre d'échantillonnage (104) ; éventuellement, dans lequel le mécanisme de mouvement comprend un moteur pas à pas et/ou un dispositif de positionnement de bande de capteur.

4. Système selon l'une quelconque des revendications précédentes, dans lequel la chambre d'échantillonnage (104) comporte une position ouverte et une position fermée, de préférence dans lequel la chambre d'échantillonnage (104) est un tiroir et le tiroir est mobile de la position ouverte à la position fermée ; et/ou
dans lequel l'appareil (102) comprend en outre un interrupteur, dans lequel l'actionnement de l'interrupteur met la source d'alimentation sous tension, de préférence dans lequel l'interrupteur est actionné lorsque la chambre d'échantillonnage (104) est dans la position fermée ; et/ou dans lequel la source d'alimentation est mise hors tension après une période de temps prédéterminée.

5. Système selon l'une quelconque des revendications précédentes, dans lequel l'appareil (102) est un appareil portatif.

6. Système selon l'une quelconque des revendications précédentes, dans lequel la chambre d'échantillonnage (104) comporte une capacité maximale dans une plage d'environ 10 cm³ à environ 300 cm³ ; et/ou
dans lequel la distance entre le niveau du sol lorsque la chambre d'échantillonnage (104) est remplie à sa capacité maximale, et la bande de capteur (20 ; 40), est dans la plage d'environ 0,5 cm à 5 cm, de préférence d'environ 1 cm à environ 3 cm ; et/ou
dans lequel la hauteur libre lorsque la chambre d'échantillonnage (104) est remplie à sa capacité maximale et lorsque la chambre d'échantillonnage (104) est dans la position fermée comporte un volume dans la plage d'environ 3 cm³ à environ 50 cm³.

7. Système selon l'une quelconque des revendications précédentes, dans lequel une ou chacune de la paire d'électrodes se présente sous la forme de doigts interdigités ou de spirales concentriques ; et/ou
dans lequel une ou chacune de la première électrode (26 ; 46) et/ou une ou chacune de la seconde électrode (28 ; 48) comprend un métal, un oxyde métallique, un polymère électriquement conducteur, du graphène ou du carbone, de préférence, de l'argent, de l'or, du cuivre, du zinc, du carbone, des nanoplots de graphène, de l'oxyde d'indium et d'étain, du poly(3,4-éthylènedioxythiophène) polystyrène sulfonate (PEDOT:PSS), du 3,4-éthylènedioxythiophène ou un dérivé de celui-ci ou du poly(3,4-éthylènedioxythiophène ou un dérivé de celui-ci ou toute combinaison de deux ou plus de ceux-ci ; et/ou
dans lequel la distance entre la première électrode (26 ; 46) et la seconde électrode (28 ; 48) de l'une ou de chacune de la paire d'électrodes est dans la plage d'environ 0,1 µm à environ 40 µm.

8. Système selon l'une quelconque des revendications précédentes, dans lequel une ou chacune de la première électrode (26 ; 46) et/ou une ou chacune de la seconde électrode (28 ; 48) sont imprimées, de préférence imprimées par flexographie, imprimées par gravure, imprimées en offset, sérigraphiées ou imprimées numériquement, et/ou
dans lequel un ou chacun des polymères semi-conducteurs (30 ; 62) sont imprimés.

9. Système selon l'une quelconque des revendications précédentes, dans lequel la longueur de l'une ou chacune de la première électrode (26 ; 46) et/ou de l'une ou chacune de la seconde électrode (28 ; 48) est dans la plage d'environ 1 cm à environ 50 cm ; et/ou
dans lequel la largeur et l'épaisseur de l'une ou chacune de la première électrode (26 ; 46) et/ou de l'une ou chacune de la seconde électrode (28 ; 48) sont chacune indépendamment dans la plage d'environ 3 nm à environ 1,5 µm ; et/ou
dans lequel un ou chacun des polymères semi-conducteurs (30 ; 62) est un polymère semi-conducteur organique ; et/ou
dans lequel un ou chacun des polymères semi-conducteurs (30 ; 62) comporte un poids moléculaire d'environ 10 000 à environ 500 000.

10. Système selon l'une quelconque des revendications précédentes, dans lequel au moins deux des polymères semi-conducteurs (30 ; 62) comprennent un polymère semi-conducteur différent ; et/ou
dans lequel un ou chacun des polymères semi-conducteurs (30 ; 62) comporte une épaisseur d'environ 1 nm à environ 100 µm.

11. Système selon l'une quelconque des revendications précédentes, dans lequel :
v) la bande de capteur (20 ; 40) comprend une couche de support sur une seconde surface du substrat flexible (42), dans lequel la seconde surface est opposée à la première surface (54), de préférence dans lequel la couche de support comprend du papier, du carton ou un film polymère ; et/ou
vi) dans lequel la bande de capteur (20 ; 40) comprend un cadre dans lequel le cadre est agencé sensiblement autour de l'extérieur de la bande de capteur (20 ; 40), de préférence dans lequel le cadre est amovible ; et/ou
vii) dans lequel le substrat flexible (42) comprend du polyimide, du polyéthylène, du polypropylène, du polyéthylène téréphtalate, du chlorure de polyvinyle, du polyamide, du polystyrène, du polycarbonate, du polylactide, de la cellulose, de l'amidon, du papier, du papier enduit de cire, du papier enduit de plastique, de films plastiques stratifiés, ou toute combinaison de deux ou plus de ceux-ci.

12. Système selon l'une quelconque des revendications précédentes, dans lequel le substrat flexible (42) comporte une épaisseur dans la plage d'environ 10 µm à environ 1 mm ; et/ou
dans lequel la bande de capteur (20 ; 40) comprend environ 3 à environ 20 capteurs polymères semi-conducteurs (24 ; 124) ; et/ou
dans lequel la longueur de la bande de capteur (20 ; 40) est dans la plage d'environ 1 cm à environ 12 cm ; et/ou
dans lequel la largeur de la bande de capteur (20 ; 40) est dans la plage d'environ 0,5 cm à environ 8 cm.

13. Procédé d'analyse de sol comprenant :
a) la fourniture d'un appareil (102), dans lequel l'appareil (102) comprend une chambre d'échantillonnage (104), une ouverture de bande de capteur (108) dans la chambre d'échantillonnage (104), une source d'alimentation et un détecteur de résistance électrique ; un affichage (105) et/ou un stockage de données (103) ;
b) la fourniture d'une bande de capteur de composés organiques volatils (COV) dans le sol (20 ; 40),
dans lequel la bande de capteur (20 ; 40) comprend un substrat flexible (42) avec une première surface (54) et un réseau de capteurs polymères semi-conducteurs (24 ; 124) agencés sur la première surface (54), dans lequel chacun des capteurs polymères semi-conducteurs (24 ; 124) comprend une paire d'électrodes, dans lequel la paire d'électrodes comprend une première électrode (26 ; 46) et une seconde électrode (28 ; 48), dans lequel un polymère semi-conducteur (30 ; 62) est disposé entre la première électrode (26 ; 46) et la seconde électrode (28 ; 48) ;
c) la fourniture d'un échantillon de sol,
d) le positionnement de la bande de capteur (20 ; 40) sur l'ouverture de bande de capteur (108), dans lequel un ou chacun des capteurs polymères semi-conducteurs (24 ; 124) sont en communication fluidique avec la chambre d'échantillonnage (104), dans lequel chaque paire d'électrodes est connectée électriquement à la source d'alimentation et au détecteur de résistance électrique,
e) le positionnement de l'échantillon de sol dans la chambre d'échantillonnage (104),
f) l'actionnement de la source d'alimentation pour fournir de l'électricité à la bande de capteur (20 ; 40), et
g) la détection de la résistance électrique d'un ou de chacun des capteurs polymères semi-conducteurs en utilisant le détecteur de résistance électrique pour donner une sortie ;
h) la transmission de la sortie du détecteur de résistance électrique à l'affichage (105) et/ou au stockage de données (103) ; l'envoi de la sortie pour comparaison à un ensemble de données connu et l'affichage d'une sortie de comparaison sur l'affichage (105) et/ou le stockage sur le stockage de données (103).

14. Procédé selon la revendication 13, comprenant en outre :
i) l'utilisation d'un algorithme d'apprentissage automatique pour rechercher des modèles dans la sortie ; éventuellement comprenant en outre :
j) la fourniture de conseils d'entretien du sol sur la base de l'étape i), de préférence dans lequel les conseils d'entretien du sol comprennent l'ajustement de la teneur en matière organique du sol, du niveau de pH, de la teneur en eau, de la teneur en nitrate, de la biomasse microbienne ou de la teneur en champignons, ou toute combinaison de deux ou plus de ceux-ci.
